# EUROPÄISCHE PATENTANMELDUNG

(11) **EP 4 850 813 A1**
(43) Veröffentlichungstag der Anmeldung: **09.12.2026**
(21) Anmeldenummer: 26182563.2
(22) Anmeldetag: 02.06.2026
(51) Int. Cl.: B01L 9/00

(54) **BIOPSIEHALTER FÜR DIE MIKROFLUIDISCHE LANGZEITKULTIVIERUNG UND (CHEMO-)THERAPIETESTS**

(30) Priorität: 02.06.2025 DE 202025001455 U
(71) Anmelder: Life on a Chip, 9611 JW Sappemeer (NL)
(72) Erfinder: Hochstetter, Axel, 9611 JW Sappemeer (NL)
(74) Vertreter: Keilitz & Partner Patentanwälte PartGmbB

(57) **Zusammenfassung**

Die Erfindung betrifft einen Biopsiehalter (1) mit den im Anspruch 1 genannten Merkmalen.

## Beschreibung

### Technisches Fachgebiet

Die Erfindung betrifft einen mikrofluidischen Biopsiehalter zur langfristigen Kultivierung und mikroskopischen Analyse von Gewebeproben, insbesondere Tumorbiopsien, unter dynamischen Perfusionsbedingungen. Der Halter ist für den Einsatz in Inkubatoren und Mikroskopen geeignet und ermöglicht patientenspezifische Experimente in modularen Organon-Chip-Systemen.

### Stand der Technik

Bekannte Biopsiehalter sind meist statisch aufgebaut, schwer zugänglich und oftmals inkompatibel mit Langzeitversuchen, kontinuierlicher Perfusion und hochauflösender Mikroskopie. Es fehlt an Lösungen, die standardisierte Außenabmessungen (z. B. Objektträgerformat), individuell anpassbare Geometrien für Biopsien und eine kontrollierte, leckagefreie Medienführung miteinander kombinieren. Zudem sind viele bestehende Systeme nicht wiederverschließbar, nicht langzeitdicht oder nicht für den modularen Einsatz in automatisierten Analyseplattformen geeignet.

Aus der US 2014/0113838 A1 ist ein mikrofluidisches Gerät bekannt, das zur Vorhersage der Arzneimittelwirkung auf Tumorbiopsien dient. Dieses System fokussiert jedoch auf schnelle Testdurchläufe unter einfacheren Bedingungen und weist keine wiederverschließbare oder individuell konfigurierbare Biopsieaufnahmeeinheit mit flexibler Medienführung auf.

Die WO 2021/058782 A1 beschreibt ein Probenverarbeitungssystem mit mikrofluidischer Kartusche, das zur automatisierten Analyse von Gewebeproben dient. Die Vorrichtung basiert jedoch auf geschlossenen Einwegkomponenten mit eingeschränkter Mikroskopiekompatibilität und erlaubt keine Anpassung an unterschiedliche Biopsiegeometrien oder modulare Kopplung mehrerer Gewebeeinheiten.

Weiterhin offenbart US 10,087,422 B2 sogenannte ´´Organ-on-Chip´´-Systeme, die die Funktion einzelner Organe in vitro abbilden. Diese Systeme sind allerdings primär für synthetisch erzeugte Zellkulturen ausgelegt und nicht für die direkte Aufnahme und Langzeitperfusion realer Biopsien mit individueller Geometrie konzipiert.

In der US 11,022,603 B2 wird ein mikrofluidisches System zur immunologischen Wirkstoffbewertung vorgestellt. Zwar erlaubt dieses System eine gewisse Wechselwirkungserfassung, jedoch fehlt es an einer wiederverschließbaren Struktur zur wiederholten Zugänglichkeit der Probe.

Die US 9,149,806 B2 betrifft mikrofluidische Geräte zur Zellseparation und ist hinsichtlich ihrer technischen Zielsetzung und des Aufbaus nicht vergleichbar mit einem Halter zur Langzeitkultivierung und mikroskopischen Beobachtung von biopsiebasiertem Gewebe.

### Aufgabe der Erfindung

Es ist daher eine Aufgabe der Erfindung, einen neuartigen Halter zur Langzeitkultivierung und mikroskopischen Beobachtung von Biopsien zu schaffen.

Gelöst wird diese Aufgabe gemäß der Erfindung durch die im Anspruch 1 angegebenen Merkmale. Weitere Ausgestaltungen der Erfindung ergeben sich aus den Unteransprüchen.

### Zusammenfassung der Erfindung

Die vorliegende Erfindung geht über diese bekannten Lösungen hinaus, indem sie ein flexibel konfigurierbares, biokompatibles und steril wiederverwendbares System bereitstellt, das speziell auf die Erfordernisse der Langzeitkultivierung realer Patientenproben unter mikrofluidischen Bedingungen und deren kontinuierlicher mikroskopischer Analyse zugeschnitten ist. Darüber hinaus erlaubt das System die Kombination mehrerer Gewebeeinheiten - etwa Tumor-, Leber- und Nierenproben - in einem verbundenen modularen Aufbau zur Untersuchung patientenspezifischer Wechselwirkungen und Metabolisierungseffekte.

Entsprechend wird gemäß der Erfindung ein Biopsiehalter zur Aufnahme mindestens einer Gewebeprobe unter mikrofluidischen Bedingungen vorgeschlagen, umfassend:
- ein mehrteiliges Gehäuse, welches eine Aufnahmekammer für die Gewebeprobe bildet, wobei die Aufnahmekammer aus wenigstens zwei Teilen zusammengesetzt ist;
- mindestens einen Einlass und einen Auslass zum Durchleiten eines kontinuierlichen oder intermittierenden Perfusionsflusses eines Kulturmediums durch die Aufnahmekammer;
- eine Dichtvorrichtung zum Abdichten der Aufnahmekammer, die z.B. in Form eines integrierten Silikon-O-Rings, X-Rings oder Dichtbandes realisiert sein kann.

Das Gehäuse des Biopsiehalters ist vorzugsweise aus einem biokompatiblen, sterilisierbaren Material (z.B. aus Poly(ethylen glykol)diacrylat [PEGDA] oder anderen Polymeren) hergestellt und kann z.B. mittels 3D-Druck gefertigt sein.

Der Einlass und Auslass befinden sich vorzugsweise an der Ober- oder Unterseite des Biopsiehalters im Abstand zur Aufnahmekammer.

Der Biopsiehalter umfasst vorzugsweise ein Unterteil und ein Oberteil, die jeweils einen Teil der Aufnahmekammer bilden. Gemäß einer Ausführungsform der Erfindung ist das Oberteil als ein Deckel ausgeführt, der in das Unterteil eingesetzt wird.

Die Dichtvorrichtung zum Abdichten der Aufnahmekammer kann z. B. als Ringdichtung realisiert sein, die in eine in der Umfangsfläche des Oberteils ausgebildeten Nut eingesetzt ist.

Außerdem kann eine Rastvorrichtung - wie z. B. Rastvorsprünge - vorgesehen sein, mit der das Oberteil mit dem Unterteil verrastet werden kann.

In einer speziellen Ausführungsform der Erfindung ist eine Klebefolie auf die Oberfläche des Biopsiehalters über das Oberteil geklebt. Die Klebefolie verhindert insbesondere, dass das Oberteil ungewollt abfällt und bietet eine zusätzliche Abdichtung.

Die Aufnahmekammer ist vorzugsweise individuell an die Biopsie-Geometrie angepasst.

Das Gehäuse des Biopsiehalters hat vorzugsweise die Außenmaße (LxB) eines standardisierten Objektträgers. (75 mm x 25 mm - 76mmx 26 mm) oder von standardisierten Mikrotiterplatten 127,76 mm × 85,48 mm × 14,35 mm (Länge × Breite × Höhe).

Der erfindungsgemäße Biopsiehalter umfasst vorzugsweise eine integrierte transparente Bodenfläche, insbesondere eine Glasplatte, zur hochauflösenden

Mikroskopie. Die transparente Bodenfläche ist vorzugsweis in einer im Gehäuse vorgesehenen Aussparung angeordnet. Die Glasplatte befindet sich vorzugsweise auf einer dem Einlass und Auslass gegenüberliegenden Seite des Biopsiehalters. Einlass und Auslass können sich z. B. an einer Oberseite, und die Glasplatte auf einer Unterseite des Biopsiehalters befinden.

Gemäß einer bevorzugten Ausführungsform der Erfindung hat die Aufnahmekammer auf ihrer Innenseite eine spezielle Struktur welche die Biopsie passgenau an Ort und Stelle festhält, ohne sie zu beschädigen. Die Struktur der Aufnahmekammer ist vorzugsweise derart dimensioniert, dass sie die jeweilige Biopsie an mehreren Stellen berührt und festklemmt, ohne sie zu beschädigen. Die Struktur ist so offen gestaltet, dass die Biopsie rundherum mit (Nähr-/ Zellkultur-)Medium umspült werden kann, um einen konstanten und großflächigen Austausch von Nährstoffen und eine Abfuhr von Abfallstoffen gewährleisten zu können. Die Struktur kann z. B. (Hohl-)Rippen oder Stege aufweisen.

Der Einlass und Auslass sind vorzugsweise über einen Kanal sowie die Aufnahmekammer fluidtechnisch miteinander verbunden. Der Kanal ist vorzugsweise im Material des Biopsiehalters integriert. Er kann z. B. hergestellt werden, indem das Material beim 3D-Druck dort ausgespart wird. In einer speziellen Ausführungsform umfasst der Biopsiehalter mehrere Aufnahmekammern. Die Aufnahmekammern können in separaten Kreisläufen integriert oder fluidisch über Kanäle miteinander verbunden sein

Der erfindungsgemäße Biopsiehalter ermöglicht die langfristige Kultivierung und Untersuchung von Gewebeproben - insbesondere Tumorbiopsien - unter mikrofluidischen Bedingungen. Ziel ist es, die Wirkung unterschiedlicher Medikamente direkt am Patientenmaterial zu testen, um so eine individualisierte, optimierte Therapieentscheidung zu treffen. Die Erfindung erlaubt eine kontrollierte Beobachtung der Zellreaktionen in Echtzeit - sowohl auf Zielzellen (z. B. Tumorzellen) als auch auf gesunde Zelltypen des gleichen Patienten - und unterstützt damit die frühe Bewertung therapeutischer Wirkung und potenzieller Nebenwirkungen.

Die Biopsie kann auch durch ein aus patienteneigenen Zellen generiertes Organäquivalent ersetzt werden, z. B. Leberorganoide, Nierenorganoide oder andere Zellkulturen. Diese werden durch die Geometrie der Aufnahmekammer sowie die individuelle Fertigungstechnologie unterstützt.

Mehrere Halter können fluidisch und mechanisch in einem separaten modularen System verbunden werden, z. B. mittels standardisierter Kupplungselemente, um komplexe metabolische und systemische Wirkzusammenhänge darzustellen, einschließlich der Verstoffwechslung von Medikamenten in einem Organmodul (z. B. Leber) und der Wirkung der Metaboliten auf Zielgewebe (z. B. Tumor).

Die Module können so konfiguriert werden, dass sie in einem gemeinsamen mikrofluidischen Kreislauf betrieben werden oder individuell geschaltet sind (parallele oder serielle Konfiguration). Dadurch kann ein ´´Life-Chip´´- oder "BioChip"-System entstehen, welches verschiedene Aspekte der individuellen Physiologie (z. B. Leber-, Nieren- und Tumorreaktion) simultan abbildet.

Durch die Kombination aus individueller Geometrieanpassung, mikrofluidischer Steuerung und modularer Verknüpfbarkeit können in vitro Bedingungen geschaffen werden, die Untersuchungen zu physiologisch realitätsnahen Reaktionen des Patientenmaterials auf Therapieansätze ermöglichen. Die optische Zugänglichkeit durch integrierten Glasboden oder beschichtbare, transparente Flächen erlaubt kontinuierliche, hochauflösende Beobachtung zellulärer Reaktionen - insbesondere durch Fluoreszenz- oder Konfokalmikroskopie.

Die wiederverwendbare, hermetisch dichtbare Konstruktion erlaubt Langzeitversuche (z.B. 2 bis 14 Tage) unter Inkubatorbedingungen ohne Medienleckage oder Kontaminationsrisiko, ermöglicht kontrollierte mikrofluidische Experimente mit hoher Reproduzierbarkeit und fördert personalisierte Medizin durch standardisierte Integration in automatisierte Analyseprozesse.

### Kurze Beschreibung der Zeichnungen

Die Erfindung wird nachstehend anhand der beigefügten Zeichnungen beispielhaft näher erläutert. Es zeigt:

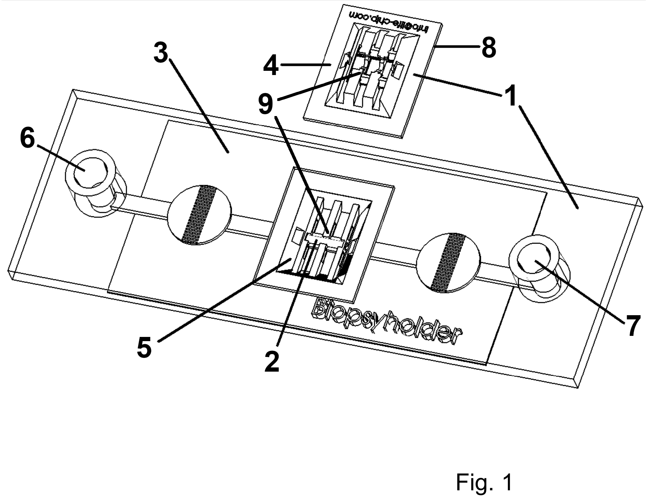

Fig. 1 einen zweiteiligen Biopsiehalter gemäß einer Ausführungsform der Erfindung.

Figur 1 zeigt einen Biopsiehalter 1, der im Wesentlichen aus einem Oberteil 4 und einem Unterteil 3 besteht. In dem Unterteil 3 ist ein durchsichtiger Boden, hier ein Glasboden, integriert. Im zusammengesetzten Zustand bilden das Unterteil 3 und das Oberteil 4 eine Aufnahmekammer 5 zur Aufnahme einer Biopsie 2.

Das Unterteil 3 ist - mit Ausnahme des Glasbodens - vorzugsweise im 3D-Druckverfahren hergestellt. Die Aufnahmekammer 5 hat auf ihrer Innenseite eine spezielle Struktur 9 welche die Biopsien passgenau an Ort und Stelle festhält, ohne sie zu beschädigen. Die Struktur 9 der Aufnahmekammer 5 berührt die jeweilige Biopsie an mehreren Stellen und hält diese fest, ist jedoch so offen gestaltet, dass die Biopsie nicht beschädigt wird, jedoch rundherum mit (Nähr-/ Zellkultur-)Medium umspült werden kann, um einen konstanten und großflächigen Austausch von Nährstoffen und eine Abfuhr von Abfallstoffen gewährleisten zu können. Hierzu kann die Aufnahmekammer 5 z. B. (Hohl-)Rippen oder Stege oder Stempel oder andere Abstandshalter aufweisen, die die Biopsie im Abstand zur Innenwand der Aufnahmekammer 5 halten.

Das Unterteil 3 umfasst außerdem einen Hauptkanal, welcher den Einlass 6 und den Auslass 7 verbindet und das durch den Einlass 6 zugeführte Medium zur Biopsie zuführt und von dort via den Auslass 7 abführt. Das Medium kann z. B. mittels einer Pumpe oder Spritze zugeführt werden.

Entlang dieses Hauptkanals können weitere Strukturen angebracht sein, um Beispielsweise Gasblasen abzufangen (sog. Bubbletraps). Die genannten Bubbletraps sind in Fig. 1 als Kreise mit Schraffur in der Mitte dargestellt.

Das Oberteil 4 bzw. Deckel enthält eine ähnliche und teilweise komplementäre Struktur 9 wie das Unterteil 3, die die Biopsie festhält und gleichzeitig ein Umströmen mit Medium erlaubt. Das Umströmen ist ein biologisch wichtiger Vorgang, um die Langlebigkeit der Biopsie außerhalb des Körpers zu gewährleisten.

Das Oberteil 4 kann mit dem Unterteil 3 verbunden werden um die Biopsie darin zu versiegeln, womit die Biopsie nur noch mit dem sie umspülenden Medium Stoffaustausch betreiben kann, aber nicht mehr mit der weiteren Umgebung. Dazu sind Oberteil 4 und Unterteil 3 aus einem gasdichten Material gedruckt. Das Oberteil 4 weist eine umfangseitige Nut auf, in die ein Silikon-O-Ring oder Ähnliches eingesetzt werden kann, um ein gas- und flüssigkeitsdichtes Versiegeln zu gewährleisten.

Das Oberteil 4 kann zudem mittels einer Klebefolie mit dem Unterteil 3 reversibel verklebt werden, um das Medium mit Überdruck durch den Biopsiehalter 1 zu drücken.

Sowohl Unterteil 3 als auch Oberteil 4 können (müssen aber nicht) symmetrisch sein; der Auslass 7 kann auch als Einlass 6 benutzt werden und umgekehrt.

Es lassen sich mehrere Biopsiehalter 1 miteinander verbinden um die Stoffwechselprodukte einer Biopsie einer anderen Biopsie zuzuführen (z.B. Krebsgewebe und Lymphgewebe) und auch Feedback-Reaktionen zwischen mehreren Biopsien zu provozieren und zu messen.

### Bezugszeichenliste

1 Biopsiehalter
2 Gewebeprobe
3 Unterteil
4 Oberteil
5 Aufnahmekammer für die Gewebeprobe
6 Einlass
7 Auslass
8 Dichtvorrichtung
9 Struktur

### Zitierte Patentliteratur

US 2014/0113838 A1
WO 2021/058782 A1
US 10,087,422 B2
US 11,022,603 B2.
US 9,149,806 B2.

## Patentansprüche

1. Biopsiehalter (1) zur Aufnahme mindestens einer Gewebeprobe (2) unter mikrofluidischen Bedingungen, umfassend:
• ein mehrteiliges Gehäuse (3, 4), welches eine Aufnahmekammer (5) für die Gewebeprobe bildet;
• mindestens einen Einlass (6) und einen Auslass (7) zum Durchleiten eines Kulturmediums durch die Aufnahmekammer (5);
• eine Dichtvorrichtung (8) zum Abdichten der Aufnahmekammer (5);
wobei das Gehäuse (3, 4) aus einem biokompatiblen, sterilisierbaren Material hergestellt ist.

2. Biopsiehalter nach Anspruch 1, **dadurch gekennzeichnet, dass** er wenigstens ein Unterteil (3) und ein Oberteil (4) umfasst, die im zusammengesetzten Zustand die Aufnahmekammer (5) bilden.

3. Biopsiehalter nach Anspruch 2, **dadurch gekennzeichnet, dass** das Unterteil (3) und/oder das Oberteil (4) eine Struktur 9 aufweisen, die die Gewebeprobe (2) festhält und gleichzeitig ein Umströmen mit dem Medium erlaubt.

4. Biopsiehalter nach Anspruch 1, **dadurch gekennzeichnet, dass** eine Klebefolie über das Oberteil (4) geklebt ist.

5. Biopsiehalter nach einem der vorhergehenden Ansprüche, **dadurch gekennzeichnet, dass** die Aufnahmekammer (5) individuell an die Biopsiegeometrie angepasst ist.

6. Biopsiehalter nach einem der vorhergehenden Ansprüche, **dadurch gekennzeichnet, dass** das Gehäuse (3, 4) die Außenmaße, insbesondere Länge und Breite, eines Standard-Objektträgers oder einer Mikrotiterplatte aufweist.

7. Biopsiehalter nach einem der vorhergehenden Ansprüche, **dadurch gekennzeichnet, dass** er eine integrierte transparente Bodenfläche, insbesondere eine Glasplatte zur hochauflösenden Mikroskopie, enthält.

8. Biopsiehalter nach Anspruch 7, **dadurch gekennzeichnet, dass** der Einlass (6) und der Auslass (7) auf einer Oberseite des Biopsiehalters (1) und die transparente Bodenfläche auf einer Unterseite des Biopsiehalters (1) angeordnet sind.

9. Biopsiehalter nach einem der vorhergehenden Ansprüche, **dadurch gekennzeichnet, dass** mehrere Aufnahmekammern (5) mit jeweils getrennten mikrofluidischen Kreisläufen vorhanden sind.

10. Biopsiehalter nach Anspruch 9, **dadurch gekennzeichnet, dass** die Aufnahmekammern (5) mikrofluidisch miteinander verbunden sind.

11. Biopsiehalter nach einem der vorhergehenden Ansprüche, **dadurch gekennzeichnet, dass** mehrere Biopsiehalter (1) mechanisch und fluidisch zu einem Modul verbindbar sind.
